# EUROPÄISCHE PATENTSCHRIFT

(11) **EP 0 315 138 B1**
(45) Veröffentlichungstag und Bekanntmachung des Hinweises auf die Patenterteilung: **27.01.1993**
(21) Anmeldenummer: 88118234.9
(22) Anmeldetag: 02.11.1988
(51) Int. Cl.: C07D 265/26

(54) **Verfahren zur Herstellung von 6-Chorisatosäureanhydrid**
Process for the preparation of 6-chloroisatoic anhydride
Procédé de préparation d'anhydride 6-chloroisatoique

(30) Priorität: 05.11.1987 DE 3737497
(43) Veröffentlichungstag der Anmeldung: 10.05.1989
(73) Patentinhaber: HOECHST AKTIENGESELLSCHAFT, 65926 Frankfurt am Main (DE)
(72) Erfinder: Folz, Georg, Dr-, D-6000 Frankfurt am Main 71 (DE); Papenfuhs, Theodor, Dr., D-6000 Frankfurt am Main 70 (DE)

(56) Entgegenhaltungen:
- DE-A- 2 206 863
- SYNTHESIS, Nr. 7, Juli 1980, Seiten 513-514, Georg Thieme Verlag; G.M. COPPOLA: "The chemistry of isatoic anhydride"

## Beschreibung

Gegenstand der vorliegenden Erfindung ist ein Verfahren zur Herstellung von 6-Chlorisatosäureanhydrid in hoher Ausbeute und Reinheit ohne zusätzliche Reinigungsoperationen durch Umsetzung von Isatosäureanhydrid, suspendiert in Eisessig oder Chloressigsäuren bzw. deren Gemischen, mit Chlorgas.

6-Chlorisatosäureanhydrid ist ein wertvolles Vor- und Zwischenprodukt zur Herstellung von Farbstoffen, Pharmazeutika und Pflanzenschutzmitteln.

Im allgemeinen gewinnt man 6-Chlorisatosäureanhydrid durch Oxidation von 5-Chlorisatin mit Chromsäure in Essigsäure (J. pr. Ch. 33 (1886), 44; J. Am. Chem. Soc. 60 (1938), 1411; J. Chem. Soc. (1957), 2916) oder durch Umsetzung von Chloranthranilsäure mit Phosgen (J. Am. Chem. Soc. (1961), 613) oder Chlorameisensäureester. Bekannt ist auch, nach der US-Patentschrift 3324119 und der Österreichischen Patentschrift 269872 Monochlorphthalimid mit Natriumhypochlorit in alkalischem Milieu zu Chlorisatosäureanhydrid umzusetzen. Diese bekannten Verfahren sind sehr ineffektiv. So lassen sich die benötigten Ausgangsprodukte beispielsweise nur umständlich und unter hohen Reinigungsverlusten aus Gemischen mit den entsprechenden isomeren Monochlor- und Dichlorderivaten erhalten.

Nach der DE-OS 2206863 kann Chlorisatosäureanhydrid hergestellt werden, indem man Isatosäureanhydrid in Lösungsmitteln, wie Benzol, Nitrobenzol oder Chlorbenzolen, in Anwesenheit von Stickstoffverbindungen, wie Dimethylformamid oder Pyridin, mit Sulfurylchlorid umsetzt, wobei Ausbeuten von 83 bis 95 % bei mäßigen Reinheiten (Schmelzpunkt nur 256 bis 262°C) erhalten werden. Für die technische Durchführung ist dieses Verfahren u.a. aufgrund des Einsatzes hygienisch bedenklicher und umweltschädlicher Lösungs- und Chlorierungsmittel, über deren einfache Rückführbarkeit nichts ausgesagt ist, weniger geeignet.

Die Chlorierung von Isatosäureanhydrid mit Chlor wird im J. pr. Ch. 33 (1886), 44 beschrieben, die jedoch nur zu einem nicht trennbaren Gemisch aus Ausgangsmaterial und wenig 6-Chlorisatosäureanhydrid führt. Weiter wird eine derartige Chlorierung in Essigsäure ohne jede detaillierte Angabe in Synth. 12 (1980) 514 erwähnt.

Es wurde nun gefunden, daß man 6-Chlorisatosäureanhydrid in hoher Ausbeute und in fast 100 %iger Reinheit durch Einleiten von Chlorgas in Suspensionen von Isatosäureanhydrid in Eisessig, Monochlor-, Dichlor- oder Trichloressigsäure oder deren beliebigen Gemischen, gegebenenfalls unter Zusatz von geringen Mengen Jod als Reaktionsbeschleuniger, bei Temperaturen von etwa 50 bis etwa 118°C, vorzugsweise von etwa 70 bis etwa 80°C, Abfiltrieren des angefallenen Produkts und ausgiebiges Waschen des Filterguts mit Wasser herstellen kann.

Die Einleitung des Chlorgases erfolgt üblicherweise bei Normaldruck. Es ist jedoch auch möglich, das Chlorgas unter Überdruck einzuleiten.

Eisessig oder Chloressigsäuren bzw. deren Gemische werden in solchen Mengen eingesetzt, daß immer eine gut rühr- und fließfähige Suspension des Isatosäureanhydrids respektive des entstandenen 6-Chlorisatosäureanhydrids vorliegt. Im allgemeinen kommt die etwa 4- bis etwa 10-fache, bevorzugt die 5- bis 7-fache Gewichtsmenge, bezogen auf die Ausgangsverbindung, in Betracht.

Die Reaktionstemperatur kann sich innerhalb eines Bereichs von etwa 50°C bis etwa 118°C (Siedepunkt der Essigsäure) bewegen; zweckmäßigerweise wählt man die Temperaturen jedoch zwischen etwa 70 und etwa 80°C, weil dann einerseits bereits eine genügende Reaktionsgeschwindigkeit herrscht und andererseits nur wenig Lösungsmittel durch den Abgasstrom mit ausgetragen wird. Die genannte obere Temperaturgrenze von etwa 118°C soll auch dann eingehalten werden, wenn man nur eine Chloressigsäure oder nur Mischungen aus Chloressigsäuren als Reaktionsmedium verwendet.

Die theoretisch benötigte Mindestmenge an Chlor ist 1 Mol pro Mol Isatosäureanhydrid. Praktisch benötigt man jedoch etwas mehr, weil in geringem Umfang die als Medium eingesetzte Essigsäure chloriert wird, was für die Reaktion völlig unschädlich ist, und ggf. etwas Chlor durch den Abgasstrom mit ausgetragen wird.

Die pro Zeiteinheit einzuleitende Chlormenge ist nicht kritisch und kann sich innerhalb eines weiten Bereiches bewegen. Bevorzugt wird eine Menge von 4 bis 5 l Chlor pro Stunde und Mol Ausgangsverbindung eingegast. Der Chlorumsatz liegt dann zu Beginn zwischen 70 und 90 % und sinkt bis zum Schluß auf ca. 10 bis 20 % ab (es findet immer auch etwas Chlorierung der Essigsäure statt, was allerdings nicht stört, da auch die resultierenden Chloressigsäuren sich als Reaktionsmedium eignen). Durch Zusatz von Jod in der Größenordnung von 0,1 bis 1 Gew.-%, bevorzugt 0,3 bis 0,5 Gew.-%5, bezogen auf Ausgangsprodukt, setzt der Umsatz bei hohen Niveau ein und wird lange dort gehalten. Das chlorwasserstoffhaltige Abchlor kann beispielsweise einer üblichen Chlorierung von aromatischen Kohlenwasserstoffen zugeführt und so vollständig und umweltfreundlich ausgenutzt werden. Die Umsetzung ist beendet, wenn eine mit Wasser behandelte und getrocknete Probe einen Schmelzpunkt von 269 bis 272°C zeigt. Chloreinleitung darüber hinaus ist nicht schädlich, da die Reaktion unter den erfindungsgemäßen Bedingungen auf der Monochlorstufe stehen bleibt.

Das erfindungsgemäße Verfahren wird zweckmäßigerweise im einzelnen so durchgeführt, daß Isatosäureanhydrid in Eisessig oder Chloressigsäuren bzw. deren Gemischen im Rahmen obengenannter Mengen suspendiert, die Mischung auf Reaktionstemperatur gebracht und Chlorgas bis zur Beendigung des Umsatzes eingeleitet wird. Die Isolierung des Reaktionsproduktes erfolgt durch Filtration. Das Filtrat, das überwiegend aus Essigsäure und Chloressigsäuren oder deren Gemischen besteht, ist ohne weitere Maßnahmen wieder einsetzbar, so daß man praktisch mit einer Erstausstattung an diesen Lösungsmitteln auskommt. Das isolierte 6-Chlorisatosäureanhydrid muß noch mit Wasser behandelt werden, was zweckmäßigerweise durch eine entsprechende Wäsche auf dem Filter erfolgt. Das Produkt enthält praktisch keine Anteile an nicht umgesetztem Isatosäureanhydrid oder isomeren oder mehrfach chlorierten Isatosäureanhydriden.

Im Vergleich zu den bekannten Verfahren und zum Stand der Technik liefert das erfindungsgemäße Verfahren in einfacher und wirtschaftlicher Weise 6-Chlorisatosäureanhydrid in hoher Ausbeute und so hoher Reinheit daß eine aufwendige Reinigung sich erübrigt. Die Reaktion erfordert nur den Einsatz der allgemein zugänglichen, kostengünstigen Essigsäure bzw. Chloressigsäuren, durch deren universelle Rückführbarkeit eine weitestgehende Schonung von Umwelt und Ressourcen erreicht wird. Zusätzliche Kosten für Beschaffung, Aufarbeitung oder Entsorgung entfallen dadurch.

In den nachfolgenden Beispielen wird das erfindungsgemäße Verfahren erläutert, ohne darauf beschänkt zu werden; Prozentangaben beziehen sich auf das Gewicht.

### Beispiel 1

163,1 g (1 mol) Isatosäureanhydrid werden in 1000 g Eisessig suspendiert und auf 75 bis 80°C erhitzt. Unter Rühren leitet man pro Stunde 4 l Chlorgas so lange ein, bis eine mit Wasser versetzte und getrocknete Probe einen Schmelzpunkt > 270°C zeigt. Die Chlorierung nimmt ca. 12 bis 14 Stunden in Anspruch. Das mit Chlorwasserstoff vermische Abchlor wird einer üblichen Aromatenchlorierung zugeführt und dort vollständig umgesetzt. Nach Abkühlung wird abgesaugt. Das Filtrat ist ohne weitere Behandlung für eine Folgechlorierung wieder einsetzbar. Das Filtergut wird ausgiebig mit Wasser gewaschen und gegebenenfalls getrocknet.

Man erhält 188 g 6-Chlorisatosäureanhydrid vom Schmelzpunkt 270 bis 270°C (Zers.), was einer Ausbeute von 95 % der Theorie entspricht. Nach HPLC-Analyse ist der Reinheitsgrad > 99 %.

### Beispiel 2

Setzt man statt Eisessig die gleiche Menge einer Mischung aus gleichen Teilen Essigsäure, Chloressigsäure und Dichloressigsäure ein und verfährt im übrigen wie in Beispiel 1 beschrieben, so erhält man das 6-Chlorisatosäureanhydrid in gleicher Reinheit und Ausbeute wie gemäß Beispiel 1.

### Beispiel 3

Bei gleichem Verfahren und gleichen Einsatzmengen, wie in Beispiel 1 beschrieben, nur unter Zusatz von 0,5 Jodg ist die Chlorierung bereits nach 8 bis 10 Stunden bei gleicher Ausbeute und Reinheit, wie nach Beispiel 1 erhalten, abgeschlossen.

### Beispiel 4

Setzt man anstelle frischen Eisessigs jeweils die vom vorhergegangenen Ansatz zurückerhaltene Essigsäure unter Ergänzung des Filtrationsverlustes auf 1000 g ein und verfährt im übrigen gemäß Beispiel 1, so erhält man die gleichen Mengen 6-Chlorisatosäureanhydrid in gleicher Reinheit wie dort.

Die Filtratrückführung wurde 15 mal ohne bemerkbaren Qualitätsverlust durchgeführt.

## Patentansprüche

1. Verfahren zur Herstellung von 6-Chlorisatosäureanhydrid in hoher Ausbeute und sehr hoher Reinheit, dadurch gekennzeichnet, daß man Chlorgas in Suspensionen von Isatosäureanhydrid in Eisessig, Monochlor-, Dichlor- oder Trichloressigsäure oder deren beliebigen Gemischen bei Temperaturen von etwa 50 bis etwa 118°C bis zur quantitativen Umsetzung einleitet, das angefallene Produkt abfiltriert und das Filtergut ausgiebig mit Wasser wäscht.

2. Verfahren nach Anspruch 1, dadurch gekennzeichnet, daß man in Gegenwart geringer Mengen Jod als Reaktionsbeschleuniger chloriert.

3. Verfahren nach mindestens einem der Ansprüche 1 und 2, dadurch gekennzeichnet, daß man das Isatosäureanhydrid suspendiert in der etwa 4- bis etwa 10-fachen Gewichtsmenge Essigsäure, Chloressigsäure oder deren Gemischen umsetzt.

4. Verfahren nach mindestens einem der Ansprüche 1 bis 3, dadurch gekennzeichnet, daß man bei Temperaturen von etwa 70 bis etwa 80°C umsetzt.

5. Verfahren nach mindestens einem der Ansprüche 1 bis 4, dadurch gekennzeichnet, daß man 4 bis 5 l Chlorgas pro Stunde und mol Isatosäureanhydrid einleitet.

## Claims

1. A process for the preparation of 6-chloroisatoic anhydride in high yield and very high purity, which comprises passing chlorine gas into suspensions of isatoic anhydride in glacial acetic acid, monochloro-, dichloro- or trichloroacetic acid or arbitrary mixtures thereof, at temperatures of about 50 to about 118°C, until conversion is quantitative, filtering off the resulting product, and washing the filtered material copiously with water.

2. The process as claimed in claim 1, wherein chlorination is carried out in the presence of small amounts of iodine to increase the rate of reaction.

3. The process as claimed in at least one of claims 1 and 2, wherein the isatoic anhydride is reacted in suspension in about 4 to about 10 times the amount by weight of acetic acid, chloroacetic acid or mixtures thereof.

4. The process as claimed in at least one of claims 1 to 3, wherein the reaction is carried out at temperatures of about 70 to about 80°C.

5. The process as claimed in at least one of claims 1 to 4, wherein 4 to 5 l of chlorine gas are passed in per hour and mol of isatoic anhydride.

## Revendications

1. Procédé pour préparer l'anhydride 6-chloroisatoïque avec un rendement élevé et une très grande pureté, caractérisé en ce que l'on injecte du chlore gazeux dans des suspensions d'anhydride isatoïque dans de l'acide acétique glacial, de l'acide monochlor-, dichlor- ou tri-chloracétique ou n'importe lequel de leurs mélanges, à des températures comprises entre environ 50 et 118 °C, jusqu'à ce que la réaction soit quantitative, on sépare par filtration le produit formé et on lave abondamment à l'eau le produit ainsi séparé par filtration.

2. Procédé selon la revendication 1, caractérisé en ce que l'on chlore en présence de faibles quantités d'iode faisant office d'accélérateur de réaction.

3. Procédé selon au moins une des revendications 1 et 2, caractérisé en ce que l'on fait réagir l'anhydride isatoïque en suspension dans environ 4 à 10 fois sa quantité en poids d'acide acétique, d'acide chloracétique ou de leurs mélanges.

4. Procédé selon au moins une des revendications 1 à 3, caractérisé en que l'on fait réagir à des températures comprises entre environ 70 et 80 °C.

5. Procédé selon au moins une des revendications 1 à 4, caractérisé en que l'on injecte 4 à 5 litres de chlore gazeux par heure et par mole d'anhydride isatoïque.
